(19) Europäisches Patentamt
European Patent Office
Office européen des brevets

(11) Publication number: 0 151 942
B1

(12) EUROPEAN PATENT SPECIFICATION

(45) Date of publication of patent specification: 12.04.89

(51) Int. Cl.⁴: C 07 D 233/74, A 61 K 31/415

(21) Application number: 85100336.8

(22) Date of filing: 15.01.85

# (54) Diphenyl hydrantoin silver complex and uses thereof.

(30) Priority: 16.01.84 US 570800

(43) Date of publication of application:
21.08.85 Bulletin 85/34

(45) Publication of the grant of the patent:
12.04.89 Bulletin 89/15

(84) Designated Contracting States:
AT BE CH DE FR GB IT LI LU NL SE

(56) References cited:
FR-A-1 389 841
US-A-3 223 729

(73) Proprietor: Lasker, Sigmund E.
Rivercross Roosevelt Island
New York New York 10044 (US)

(72) Inventor: Lasker, Sigmund E.
Rivercross Roosevelt Island
New York New York 10044 (US)

(74) Representative: Vossius & Partner
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 151 942 B1

## Description

The compound 5,5-Diphenyl-2,4-imidazolidinedione, also known as diphenyl hydantoin, or phenytoin, possesses the structure

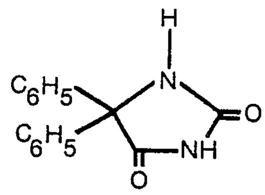

and has been used as an anticonvulsant. It is still used in this way, although it has been supplanted, to some degree, by other drugs, especially the hypnotics. See, e.g., F. Buchtal et al., *Antileptic Drugs,* Raven Press, N.Y., 1972 at page 103. It is more extensively used as a veterinary anticonvulsant, particularly in the treatment of cats.

Diphenyl hydantoin, its derivatives, and compositions containing these are known in the art, as may be seen in, e.g. U.S. Patents 2,409,754; 3,932,449; 3,798,233; 4,091,233; and 4,093,809, the disclosures of which are incorporated by reference herein. While many uses for hydantoin and its derivatives are disclosed in these references, it is apparent that hydantoin and its derivatives have never been employed as biocides.

Organometallic compounds have been employed in the past as specific biocides, particularly as antimicrobials. For example, organometallic derivatives of sulfadiazine are among the most prominent of these materials. See, e.g. U.S. Patents 3,761,590; 4,020,150; 4,049,802; 3,792,161; and 4,078,058, in which organometallic compounds of sulfadiazine are disclosed, such as silver sulfadizine, zinc sulfadiazine, and cerium sulfadiazine. The efficacy of these compounds as antimicrobials is clear from these disclosures, which are incorporated by reference herein.

While each of these complexes exhibits satisfactory effect in particular applications, no single one of these compounds, or classes of compounds, is effective against a broad range of infections and infective agents such as bacteria, viruses or plasmodia. Additionally, treatment of target infections with the known metallic complexes of, e.g., sulfadiazine, has resulted in the development of mutant strains against which previously satisfactory anti-infection agents are now less effective, or noneffective.

Hence it is an object of this invention to provide material useful as a versatile biocide for, but not limited to, bacteria, viruses or plasmodia.

It is a further object of this invention to provide a process for the production of a versatile biocide.

This object is accomplished by a diphenyl hydantoin silver complex of the form Ag(Ph), wherein Ph is 5,5-Diphenyl-2,4-imidazolidinedione, i.e., diphenyl hydantoin, or phenytoin.

The compound 5,5-Diphenyl-2,4-imidazolidinedione, i.e., diphenyl hydantoin or phenytoin is reacted with compounds containing silver ions under conditions favorable for the production of complexes of the form Ag(Ph), wherein Ph is diphenyl hydantoin. The resulting diphenyl hydantoin silver complex is useful as a biocide in the treatment of various plant and animal infections, such as bacterial infections, viral infections, fungal infections, parasite infections, and infections by insects or other pests. Optionally the diphenyl hydantoin silver complex is used in combination with inert ingredients.

The resulting diphenyl hydantoin silver complex is then applied, either topically or parenterally to plant and/or animal tissue so as to prevent or to treat an infection. Additionally, the diphenyl hydantoin silver complex may be combined with materials which are designed to prevent infestation or infection, or to relieve infection.

The fact that low toxicity toward host tissue is observed renders the silver complex suitable for use as, e.g., a topical agent in burn therapy or in the treatment of the umbilical stump of newborn infants.

In comparative tests, the diphenyl hydantoin silver complex produced results superior to those obtained using silver sulfadiazine or sodium sulfadiazine, two compounds of choice in surface wound and burn therapy.

Diphenyl hydantoin is first converted to its ammonium salt by reacting it with ammonium hydroxide under conditions favoring formation of the ammonium salt. Following formation of the ammonium salt, a source of the silver ion with which the diphenyl hydantoin is to be complexed is added. This may be, e.g. silver nitrate solution, but may be any appropriate material which contains the silver ion sought to be complexed. The source of silver ions is added under conditions favoring formation of the diphenyl hydantoin silver complex. The resulting complexes are, in general, poorly soluble, so the formation of a precipitate may be taken as a sign that the complex has formed. Silver diphenyl hydantoin, for example, is very insoluble, and precipitates out of the reaction solution almost immediately. Upon precipitation, the diphenyl hydantoin silver complex is collected, washed free of reactant and ions, and is then dried and readied for use.

The diphenyl hydantoin silver complex of the invention may be used in biocidal compositions as bactericide, viricide, fungicide, parasiticide and/or insecticide, optionally in combination with inert ingredients. The biocidal composition may be in the form of a suspension of the active ingredient, in dust or powder form, in aerosol, cream or ointment form or may be a liquid. The diphenyl hydantoin silver complex is contained in the composition in an amount of at least 0.01 µg/ml, preferably 0.01 to 40 µg/ml.

The following examples set forth uses of the diphenyl hydantoin silver complex in various situations.

The poor solubility of the diphenyl hydantoin silver complex in aqueous media, as well as its poor diffusibility in agar media, required special techniques to achieve usable results. To this end, it was necessary to quantitate action by using a doubling dilution procedure, in Miller-Hinton broth, so as to yield reproducible results.

Example 1

The effect of the diphenyl hydantoin silver complex at various concentrations upon various microorganisms was tested. For each of the microorganisms listed below, a culture was grown in Miller-Hinton broth (24 hour culture), and then diluted at a 1:100 ratio. This diluted culture was then grown for an additional two hours, after which the diphenyl hydantoin silver complex was added. Evaluation of the culture took place 18 hours after the addition of the silver phenytoin. The results are summarized in accompanying Table 1.

TABLE 1

Effect of Silver Phenytoin on Various Microorganisms

| | Minimal Inhibitory Concentration<br>Micrograms of Diphenyl Hydantoin Silver Complex per Milliliter | | | |
|---|---|---|---|---|
| Organism | 1 | 10 | 20 | 40 |
| Enterococcus Group D Strep) | − | − | + | ++ |
| Candida Albicans | − | − | + | + |
| Klebsiella | + | + | + | ++ |
| Seratia | − | + | + | + |
| Pseudomonas aeruginosa | − | − | + | + |
| Staphloccoccus aereus | − | − | + | + |

+ = inhibition.
Organisms grown in Miller-Hinton broth.

A 24 hour culture was diluted 1:100 and compound was added to a two hour culture of this dilution and evaluated at 18 hours.

Antibacterial action was also demonstrated in pure cultures of organisms on blood agar plates.

Example 2

Experiments designed to compare the effect of Sodium Sulfadiazine, Silver Sulfadiazine, and the diphenyl hydantoin silver complex were performed. The parameters of the experiments were identical to those set forth in Example 1, and the results are summarized in Table 2.

TABLE 2

| MINIMAL INHIBITORY CONCENTRATIONS | | | | | | | | | | | | | | | | | | |
|---|---|---|---|---|---|---|---|---|---|---|---|---|---|---|---|---|---|---|
| | Enterococcus | | | Candida A. | | | Klebsiella P. | | | Seratia M. | | | Pseudomonas A. | | | Strep A. | | |
| | S | SS | SP | S | SS | SP | S | SS | SP | S | SS | SP | S | SS | SP | S | SS | SP |
| Microgram of agent | | | | | | | | | | | | | | | | | | |
| 1 | − | − | − | − | − | − | − | − | + | − | − | − | − | − | − | − | − | − |
| 10 | − | − | − | − | − | − | − | − | + | − | + | + | − | + | − | − | − | − |
| 20 | − | − | + | − | − | + | − | + | + | ± | + | + | − | + | + | − | + | + |
| 40 | − | + | ++ | − | − | + | − | + | ++ | ± | + | ++ | − | + | + | + | + | + |

Organisms were grown in Miller-Hinton broth. A twenty-four hour culture was diluted 1:100. Compounds were added to two hour cultures and examined for inhibition at 18 hours. The magnitude of inhibition is estimated and indicated by +.

S : SODIUM SULPHADIAZINE
SS : SILVER SULPHADIAZINE
SP : DIPHENYL HYDANTOIN SILVER COMPLEX

Example 3

The efficacy of the diphenyl hydantoin silver complex as a plant fungicide was tested. The diphenyl hydantoin silver complex and commercial fungicide standards were ground and suspended in acetone in an amount equal to 6% of their final volume and then suspended in purified water containing 250 ppm of the surfactant TREM 014 (polyhydric alcohol esters).

In the preventive tests, which are summarized in Tables 3—6, the suspensions were sprayed to the point of run-off on the plants, and were then inoculated 24 hours later with a spore suspension of the fungus as given in Tables 3—6. The thus treated plants were then placed in a saturated humidity chamber at 20°C for 24 hours, and then in a growth room for an additional 7—12 days. Disease ratings were then made, and recorded as percent disease control.

Curative tests were conducted in a similar matter, except that inoculation with spore fungus took place 24 hours prior to application of the chemical. The results are summarized in Tables 3—6, which follow.

TABLE 3

Evaluation of diphenyl hydantoin silver complex for the control of grape downy mildew (Plasmopara viticola)[1]

| Compound | Concentration[2] | Percent Disease Control | |
|---|---|---|---|
| | | Preventive[3] | Curative[4] |
| Silver diphenyl hydantoin | 100 | 87 | 0 |
| | 20 | 49 | 0 |
| | 5 | 24 | 0 |
| Curzate® | 100 | 100 | 100 |
| | 20 | 0 | 100 |
| | 5 | 0 | 0 |
| Manzate® | 100 | 100 | 0 |
| | 20 | 80 | —[5] |
| | 5 | 40 | — |

[1]Test plants inoculated with an aqueous sporangial suspension ($1.0 \times 10^5$ sporangia/ml).

[2]ppm.

[3]Test plants inoculated 24 hours after application of chemical.

[4]Test plants inoculated 24 hours before application of chemical.

[5]Not tested.

Note: Curzate® is the registered trademark of E. I. du Pont de Nemours & Co. for formulation of 2-Cyano-N-(ethyl-amino)carbonyl)-2-(methoxyimino) acetamide

Manzate® is the registered trademark of E. I. du Pont de Nemours & Co. for formulations of Manganese ethylenebisdithio carbamate

TABLE 4

Evaluation of diphenyl hydantoin silver complex for the control of tomato late blight (Phytophthora infestans)[1]

| Compound | Concentration[2] | Percent Disease Control |
|---|---|---|
| Silver diphenyl hydantoin | 100 | 98 |
| | 20 | 95 |
| | 5 | 17 |
| Curzate® | 100 | 100 |
| | 20 | 90 |
| | 5 | 40 |
| Manzate® | 100 | 100 |
| | 20 | 90 |
| | 5 | 0 |

[1]Test plants inoculated with aqueous sporangial suspension (2.0 × $10^4$ sporangia/ml).
[2]ppm.

TABLE 5

Evaluation of diphenyl hydantoin silver complex for the control of apple scab (Venturia inaequalis)[1]

| Compound | Concentration[2] | Percent Disease Control | |
|---|---|---|---|
| | | Preventive[3] | Curative[4] |
| Silver diphenyl hydantoin | 100 | 100 | 0 |
| | 20 | 100 | 0 |
| | 5 | 60 | 0 |
| Manzate® | 100 | 100 | 0 |
| | 20 | 55 | 0 |
| | 5 | 12 | 0 |
| Baycor® | 20 | 100 | 100 |
| | 5 | 85 | 65 |
| | 1 | 15 | 40 |

[1]Test plants inoculated with aqueous conidial suspension (1.5 × $10^5$ condidia/ml).
[2]ppm.
[3]Test plants inoculated 24 hours after application of chemical.
[4]Test plants inoculated 24 hours before application of chemical.

BAYCOR® is the registered trademark of Bayer AG (Federal Republic of Germany) and Mobay Chemical Corp., Agricultural Chemicals Division, for formulations of B (1,1-Biphenyl)-4-yloxy)a (1,1-dimethylethyl)-1H-1,2,4-triazole-ethanol.

TABLE 6

Evaluation of diphenyl hydantoin silver complex for the control of peanut leafspot (Cercospora arachidicola)[1]

| Compound | Concentration[2] | Percent Disease Control | |
|---|---|---|---|
| | | Preventive[3] | Curative[4] |
| Silver diphenyl hydantoin | 100 | 78 | 60 |
| | 20 | — | 0 |
| | 5 | 16 | 0 |
| Manzate® | 100 | 85 | 0 |
| | 20 | 30 | 0 |
| | 5 | 0 | 0 |
| Baycor® | 20 | 100 | 100 |
| | 5 | 98 | 100 |
| | 1 | 77 | 92 |

[1]Test plants inoculated with aqueous conidial suspension ($6.0 \times 10^4$ condidia/ml).
[2]ppm.
[3]Test plants inoculated 24 hours after application of chemical.
[4]Test plants inoculated 24 hours before application of chemical.

The diphenyl hydantoin silver complex is effective against various infectious agents, including bacteria, viruses, fungi, parasites, and insects. It has been found, in particular, that the diphenyl hydantoin silver complex is useful against silver sensitive parasitic infections, including, but not limited to, all species of malaria-carrying plasmodia sporoza. Thus, the diphenyl hydantoin silver complex may be applied in treatment of infections of the blood caused by, e.g., parasites.

Additionally, it is found that diphenyl hydantoin compounds accumulate in tumor cells. Hence, the compound of the invention may be used as well in the delivery of, e.g., Ag, to tumor cells. The accumulated diphenyl hydantoin silver complex allows diffusion of the silver ion to the tumor cells, resulting in a gradual release of the material.

Further subject matters of the invention therefore are a material for protecting animal and/or plant tissue from infection, comprising a protective material with which has been incorporated the diphenyl hydantoin silver complex and a material for the delivery of metallic ions to tumor cells, comprising the diphenyl hydantoin silver complex.

The material for protecting animal and/or plant tissue from infection may be a wound dressing, a bandage, a protective cover sheet, a net or netting. It may also be a plant stake, a paint or a mortar.

The method of delivery of the diphenyl hydantoin silver complex will vary depending upon the type and extent of the infection being treated or prevented. Topical application may be desirable when the tissue infected exhibits a surface infection such as, e.g., a surface wound or lesion which has been infected. This topical method of application is particularly appropriate in treating plant tissue infections. Application may be accomplished parenterally as well, taking the form, e.g., of subcutaneous injection, intravenous application or intramuscular injection. Parenteral application is particularly well suited for treating animal tissue infections, such as blood or muscle tissue infections. It is also the preferred method of application when gradual release of the diphenyl hydantoin silver complex is desired, such as in the treatment of tumor cells.

Some embodiments of the diphenyl hydantoin silver complex which are suited for topical or parenteral application include, e.g., creams or ointments, wherein one or more inert ingredients are combined with the complex to aid in its delivery to plant and animal tissue. Liquid carriers may be used, such as in water dispersions, intravenous fluids, aerosols and sprays. Additional materials which may be used as sources for the diphenyl hydantoin silver complex include dusts, dusting powders, and tinctures of the compounds or compositions containing the compounds.

More extensive media may be used as well, for larger scale protection from infection or infestation. While suspensions of the diphenyl hydantoin silver complex may be used to spray plants, e.g., when run-off is not an issue, other media, such as plant stakes treated with the compound, or protective covers may be issued. Similarly, the compounds may be incorporated with or impregnate dressings such as bandages, mosquito or other protective nets. Mortars, and other building materials may also have incorporated or impregnated therein the diphenyl hydantoin silver complex. These paints, e.g., will then act to render dwellings or shelters more infection proof than previously possible.

**Claims for the Contracting States: BE CH DE FR GB IT LI LU NL SE**

1. Diphenyl hydantoin silver complex.

2. A method for the preparation of the diphenyl hydantoin silver complex of claim 1, comprising converting diphenyl hydantoin into its ammonium salt, reacting the ammonium salt with a source of silver ions and recovering the diphenyl hydantoin silver complex formed.

3. A composition useful as a biocide, comprising the diphenyl hydantoin silver complex of claim 1 in an amount sufficient to exhibit biocidic properties.

4. A composition according to claim 3, wherein said diphenyl hydantoin silver complex is combined with inert ingredients.

5. A composition according to claim 3, wherein said diphenyl hydantoin silver complex is present in quantities of at least 0.01 µg/ml.

6. A material for protecting animal and/or plant tissue for infection, comprising a protective material with which has been incorporated the diphenyl hydantoin silver complex of claim 1.

7. A material for the delivery of metallic ions to tumor cells, comprising the diphenyl hydantoin silver complex of claim 1.

**Claims for the Contracting State: AT**

1. A method for the preparation of a diphenyl hydantoin silver complex, comprising converting diphenyl hydantoin into its ammonium salt, reacting the ammonium salt with a source of silver ions and recovering the diphenyl hydantoin silver complex formed.

2. A composition useful as a biocide, comprising the diphenyl hydantoin silver complex prepared according to claim 1 in an amount sufficient to exhibit biocidic properties.

3. A composition according to claim 2, wherein said diphenyl hydantoin silver complex is combined with inert ingredients.

4. A composition according to claim 2, wherein said diphenyl hydantoin silver complex is present in quantities of at least 0.01 µg/ml.

5. A material for protecting animal and/or plant tissue from infection, comprising a protective material with which has been incorporated the diphenyl hydantoin silver complex prepared according to claim 1.

6. A material for the delivery of metallic ions to tumor cells, comprising the diphenyl hydantoin silver complex prepared according to claim 1.

**Patentansprüche für die Vertragsstaaten: BE CH DE FR GB IT LI LU NL SE**

1. Diphenylhydantoin-Silber-Komplex.

2. Verfahren zur Herstellung des Diphenylhydantoin-Silber-Komplexes nach Anspruch 1, dadurch gekennzeichnet, daß man Diphenylhydantoin in sein Ammoniumsalz umwandelt, das Ammoniumsalz mit einer Silberionen liefernden Quelle umsetzt und den gebildeten Diphenylhydantoin-Silber-Komplex gewinnt.

3. Zusammensetzung zur Verwendung als Biozid, enthaltend den Diphenylhydantoin-Silber-Komplex nach Anspruch 1, in einer Menge, die ausreicht, um biozide Eigenschaften zu zeigen.

4. Zusammensetzung nach Anspruch 3, in der der Diphenylhydantoin-Silber-Komplex mit inerten Bestandteilen kombiniert ist.

5. Zusammensetzung nach Anspruch 3, in der der Diphenylhydantoin-Silber-Komplex in Mengen von mindestens 0,01 µg/ml vorliegt.

6. Mittel zum Schutz von Tier- und/oder Pflanzengewebe vor Infektionen, enthaltend ein schützendes Mittel, das den Diphenylhydantoin-Silber-Komplex nach Anspruch 1 umfaßt.

7. Mittel für die Versorgung von Tumorzellen mit Metallionen, enthaltend den Diphenylhydantoin-Silber-Komplex nach Anspruch 1.

**Patentansprüche für den Vertragsstaat: AT**

1. Verfahren zur Herstellung eines Diphenylhydantoin-Silber-Komplexes, dadurch gekennzeichnet, daß man Diphenylhydantoin in sein Ammoniumsalz umwandelt, das Ammoniumsalz mit einer Silberionen liefernden Quelle umsetzt und den gebildeten Diphenylhydantoin-Silber-Komplex gewinnt.

2. Zusammensetzung zur Verwendung als Biozid, enthaltend den gemäß Anspruch 1 hergestellten

Diphenylhydantoin-Silber-Komplex in einer Menge, die ausreicht, um biozide Eigenschaften zu zeigen.

3. Zusammensetzung nach Anspruch 2, in der der Diphenylhydantoin-Silber-Komplex mit inerten Bestandteilen kombiniert ist.

4. Zusammensetzung nach Anspruch 2, in der der Diphenylhydantoin-Silber-Komplex in Mengen von mindestens 0,01 µg/ml vorliegt.

5. Mittel zum Schutz von Tier- und/oder Pflanzengewebe vor Infektionen, enthaltend ein schützendes Mittel, das den gemäß Anspruch 1 hergestellten Diphenylhydantoin-Silber-Komplex umfaßt.

6. Mittel für die Versorgung von Tumorzellen mit Metallionen, enthaltend den gemäß Anspruch 1 hergestellten Diphenylhydantoin-Silber-Komplex.

**Revendications pour les Etats contractants: BE CH DE FR GB IT LI LU NL SE**

1. Le complexe diphénylhydantoïne-argent.

2. Un procédé pour la préparation du complexe diphénylhydantoïne-argent selon la revendication 1, consistant à transformer la diphénylhydantoïne en son sel d'ammonium, à faire réagir le sel d'ammonium avec une source d'ions argent et à récupérer le complexe diphénylhydantoïne-argent formé.

3. Une composition utile en tant que biocide, contenant le complexe diphénylhydantoïne-argent de la revendication 1 en une quantité suffisante pour manifester des propriétés biocides.

4. Composition selon la revendication 3, dans laquelle ledit complexe diphénylhydantoïne-argent est associé à des ingrédients inertes.

5. Composition selon la revendication 3, dans laquelle ledit complexe diphénylhydantoïne-argent est présent en des quantités d'au moins 0,01 µg/ml.

6. Une substance pour la protection des tissus d'animaux et/ou de plantes contre l'infection comprenant une substance protectrice à laquelle a été incorporé le complexe diphénylhydantoïne-argent de la revendication 1.

7. Une substance destinée à délivrer des ions métalliques à des cellules tumorales, contenant le complexe diphénylhydantoïne-argent de la revendication 1.

**Revendications pour l'Etat contractant: AT**

1. Un procédé pour la préparation d'un complexe diphénylhydantoïne-argent consistant à transformer la diphénylhydantoïne en son sel d'ammonium, à faire réagir le sel d'ammonium avec une source d'ions argent et à récupérer le complexe diphénylhydantoïne-argent formé.

2. Une composition utile en tant que biocide, contenant le complexe diphénylhydantoïne-argent préparé selon la revendication 1 en une quantité suffisante pour manifester des propriétés biocides.

3. Composition selon la revendication 2, dans laquelle ledit complexe diphénylhydantoïne-argent est associé à des ingrédients inertes.

4. Composition selon la revendication 2, dans laquelle ledit complexe diphénylhydantoïne-argent est présent en des quantités d'au moins 0,01 µg/ml.

5. Une substance pour la protection des tissus d'animaux et/ou de plantes contre l'infection, comprenant une substance protectrice à laquelle a été incorporé le complexe diphénylhydantoïne-argent préparé selon la revendication 1.

6. Une substance destinée à délivrer des ions métalliques à des cellules tumorales, contenant le complexe diphénylhydantoïne-argent préparé selon la revendication 1.